# EUROPÄISCHE PATENTANMELDUNG

(11) **EP 1 649 848 A1**
(43) Veröffentlichungstag der Anmeldung: **26.04.2006**
(21) Anmeldenummer: 05108814.4
(22) Anmeldetag: 23.09.2005
(51) Int. Cl.: A61K 8/36, A61K 8/41, A61K 8/34, A61Q 9/02, A61K 8/45

(54) **Rasierhilfsmittel**

(30) Priorität: 12.10.2004 DE 102004049773
(71) Anmelder: Beiersdorf Aktiengesellschaft, 20253 Hamburg (DE)
(72) Erfinder: Maurer, Peter, 24536, Neumünster (DE); Oelrichs, Ilka, 25436, Tornesch (DE); Conzelmann, Stefanie, 21149, Hamburg (DE)

(57) **Zusammenfassung**

Seifenhaltige Öl-in-Wasser Emulsionen, die frei von Fettsäurediethanolamiden und zur Abfüllung in Aerosolverpackungen geeignet sind.
(a) 4 bis 12 Gew.% Fettsäuren mit 12 bis 18 Kohlenstoffatomen,
(b) 3 bis 8 Gew.% Trialkanolaminen sowie als weitere Komponenten,
(c) 1 bis 8 Gew.%, bevorzugt 2 bis 6 Gew.% eines nichtionischen Emulgators
(d) 0 bis 3 Gew. % eines Fettalkohols oder Fettalkoholgemisches,
(e) 0,1 bis 6 Gew.%, besonders bevorzugt 0,5 bis 5 Gew.% eines oder mehrerer Emollientien
(f) 1 bis 10, besonders bevorzugt 2 bis 5 Gew.% Treibgas mit einem Dampfdruck von 76 bis 420 kPa bei 20°C.
(g) 0 bis 1 Gew.%, bevorzugt 0,1 bis 1 Gew.% eines Cellulosederivates und
(h) 0,1 bis 4, besonders bevorzugt bis 3, besonders bevorzugt bis 2 Gew.% Glycerinpolyglycoletherisostearate, besonders bevorzugt solches mit 70 bis 110 Ethylenoxyd-Einheiten,
jeweils bezogen auf das Gesamtgewicht der Zubereitung, aufweisen.

## Beschreibung

Die vorliegende Erfindung betrifft Rasierhilfsmittel. Man unterscheidet Rasierschäume und Rasiergele. Rasierschäume werden so formuliert, dass die Aerosol enthaltende Zubereitung aus dem Packmittel aufgeschäumt entnommen werden kann. Rasierschäume von Qualität zeichnen sich durch einen feinporigen, reichhaltigen und stabilen Schaum aus.

Rasiergele dagegen werden so formuliert, dass die ebenfalls Aerosol enthaltende Zubereitung in nicht aufgeschäumter Form aus dem Packmittel entnommen wird und später auf der Haut oder in der Hand durch Verdampfen des in der Zubereitung eingeschlossenen Treibmittels durch Zuführung der Körperwärme aufschäumt.

In beiden Fällen besteht die Kunst der Formulierung darin, durch geeignete Wahl von Verdickern und Schaumstabilisatoren einen guten Kompromiss aus Schaumtextur, Gelkonsistenz, Stabilität, Sensorik zu erreichen.

Ein häufiger Inhaltsstoff solcher Zubereitungen ist Kokosfettsäureamid DEA. Diese Verbindung gehört zur Gruppe der Fettsäurediethanolamide. Er ist ein Reaktionsprodukt aus Diethanolamin (DEA), aus der Familie der Alkanolamine und freien Fettsäuren, gewonnen aus Kokosöl. Freies DEA, das nur in schlechten Qualitäten in signifikanten Mengen vorkommt, ist in der Lage in Kombination mit N-nitrosierenden Stoffen, wie z.B. Nitriten, Nitosamine zu bilden. Da diese Nitrosamine ein carcinogenes Potential zeigen, gilt es zurecht ihre Bildung zu vermeiden. Dies kann bei sachgemäßem Einsatz des Rohstoffes auch problemlos erreicht werden. Leider werden in der öffentlichen Darstellung, z.B. in Verbraucherzeitschriften, chemische Zusammenhänge oftmals ungenau oder gar falsch dargestellt. Daher können Fettsäurediethanolamide in Markenprodukten zwar eingesetzt werden, es kann aber zuweilen der Wunsch bestehen, auf ihren Einsatz zu verzichten.

In tensidischen Zubereitungen weisen Fettsäurediethanolamide den Zusatznutzen auf, als so genannte "Schaumbooster" zu wirken: gleichzeitig wird die Stabilität, Bildung und Struktur des Schaums deutlich verbessert, dass heißt mehrere Anforderungen werden gleichzeitig durch einen Rohstoff erfüllt. Daher gibt es bisher keine Alternative zu Kokosfettsäureamid DEA in Rasierzubereitungen, da die einzigartige Kombination aller Eigenschaften nicht erreicht wird.

Wünschenswert wäre einen ähnlich wirkenden Rohstoff zu finden, der die zuvor beschriebene Problematik nicht aufweist. Da dieses Problem bereits länger besteht, wurden bereits viele Ersatzstoffe für Fettsäurediethanolamide vorgestellt. Trotz dieses großen Angebots konnte bisher kein geeigneter Ersatzstoff gefunden werden, der in Rasierzubereitungen vorteilhaft eingesetzt werden kann und wie beschrieben alle Anforderungen gleichzeitig und in vergleichbarer Qualität erfüllt.

Die internationale Anmeldung WO96/09032 offenbart seifenfreie Rasierzubereitungen auf der Basis von Sarcosinaten mit einem pH-Wert im Bereich pH 5 - 7. Demgegenüber sind erfindungsgemäße Zubereitungen seifenhaltig und weisen einen pH-Wert > 7 auf.

Die Schrift US 3923970 offenbart Rasierzubereitungen, die unter anderem Polyoxyethylenoleylether mit einem HLB im Bereich 1-9 oder Lauramid DEA enthalten. Demgegenüber sind erfindungsgemäße Zubereitungen von diesen frei.

Die Schrift EP503004 offenbart Rasierzubereitungen, die unter anderem Polysiloxan/Polyether-Copolymere enthalten. Demgegenüber sind erfindungsgemäße Zubereitungen von diesen frei.

Die internationale Anmeldung WO98/33473 offenbart Rasierzubereitungen, die unter anderem polyethoxylierte Polyvinylpyrrolidone enthalten. Demgegenüber sind erfindungsgemäße Zubereitungen von diesen frei.

Die europäische Patentanmeldung 576615 offenbart selbstschäumende O/W-Rasiercremes enthaltend Wasser, Seifenkomponente, Tensid, Treibmittel, Emollientien und Schaumstabilisator aus der Gruppe der Fettalkanolamide, z.B. Lauramid DEA. Über Zubereitungen mit einem zusätzlichen Gehalt an Cellulosederivaten bei gleichzeitiger Freiheit von Fettsäurealkanolamiden wird dagegen nichts offenbart.

Davon ausgehend lag der vorliegenden Erfindung die Aufgabe zugrunde, eine Verdicker-Tensidkombination zu finden, die nicht bei der Herstellung die Entstehung von Nitrosaminen begünstigt und gleichzeitig besseres Gleiten der Rasierklinge über die Haut erlaubt.

Es hat sich für den Fachmann nicht vorhersehbar herausgestellt, dass seifenhaltige Öl-in-Wasser Emulsionen, die frei von Fettsäurediethanolamiden und zur Abfüllung in Aerosolverpackungen geeignet sind, enthaltend
(a) 4 bis 12 Gew.% Fettsäuren mit 12 bis 18 Kohlenstoffatomen,
(b) 3 bis 8 Gew.% Trialkanolaminen sowie als weitere Komponenten,
(c) 1 bis 8 Gew.%, bevorzugt 2 bis 6 Gew.% eines nichtionischen Emulgators,
(d) 0 bis 3 Gew. % eines Fettalkohols oder Fettalkoholgemisches,
(e) 0,1 bis 6 Gew.%, besonders bevorzugt 0,5 bis 5 Gew.% eines oder mehrerer Emollientien,
(f) 1 bis 10, besonders bevorzugt 2 bis 5 Gew.% Treibgas mit einem Dampfdruck von 76 bis 420 kPa bei 20°C,
(g) 0 bis 1 Gew.%, bevorzugt 0,1 bis 1 Gew.% eines Cellulosederivates und
(h) 0,1 bis 4, besonders bevorzugt bis 3, besonders bevorzugt bis 2 Gew.% Glycerinpolyglycoletherisostearate, besonders bevorzugt solches mit 70 bis 110 Ethylenoxyd-Einheiten
   jeweils bezogen auf das Gesamtgewicht der Zubereitung, aufweisen, den Mängeln des Standes der Technik abhelfen. Diese Kombinationen wirken gleichzeitig verdickend und schmierend, so dass die Rasierklinge besser über die Haut gleitet. Glycerinpolyglycoletherisostearate wirken dabei als Gelbildner und als Verdicker, Trialkanolamine fördern die Schaumbildung und helfen bei der Einstellung der Schaumstruktur, nichtionische Emulgatoren tragen zur Ausbildung eines Gleitfilms auf der Haut bei. Solcherart Zubereitungen können ein- oder mehrphasig sein, bevorzugt sind sie zweiphasig.

Es wurde weiter gefunden, dass es bevorzugt ist, wenn der oder die nichtionischen Emulgatoren einen HLB-Wert von mindestens 14 aufweisen. Dabei ist es bevorzugt, wenn der oder die Emulgatoren gewählt werden aus der Gruppe der Fettsäurealkoxylate mit 10 bis 40, besonders bevorzugt 15 bis 30 Alkoxyeinheiten. Bevorzugt ist es weiter, wenn das oder die Cellulosederivate gewählt werden aus der Gruppe HydroxypropylMethylcellulose, Hydroxyethylcellulose, Hydroxypropylcellulose. Ebenfalls ist bevorzugt, wenn als Glycerinpolyglycoletherisostearat das Glycerinpolyglycolether-90-isostearat verwendet wird. Erfindungsgemäße Zubereitungen liegen bevorzugt als aus einer Druckgaspackung entnehmbare Schäume oder alternativ als aus einer Druckgaspackung entnehmbare Rasiergele vor. Solche Gele können transparent, durchscheinend oder trübe sein. Besonders bevorzugt ist es, wenn die Druckgaspackung zwei Kammern aufweist. Die Erfindung umfasst auch Packmittel enthaltend erfindungsgemäße Zubereitungen.

Von Vorteil ist es, wenn der Ölgehalt in Gelen 0 bis 2 Gew.%, besonders bevorzugt 0,5 bis 1,5 Gew.% beträgt. Weiterhin ist es von Vorteil, wenn der Ölgehalt in Schäumen 0 bis 6 Gew.%, besonders bevorzugt 0,25 bis 1 Gew.% beträgt.

Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

Erfindungsgemäße Zubereitungen können darüber hinaus vorteilhaft noch weitere Tenside, Antioxidantien, Vitamine, Konservierungsstoffe, wasserlösliche Farbe enthalten.

### Tenside

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielsweise ―COO⁻, ―OSO₃²⁻, ―SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wässrigem Medium keine Ionen.

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate

Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,
Phosphorsäureester und Salze, wie beispielsweise Dilaureth-4 Phosphat,

Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.

sowie
Schwefelsäureester, wie
1. Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder - bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Aminoxide, wie Cocoamidopropylaminoxid,
3. Ester, die durch Veresterung von Carbonsäuren mit Ethylenoxid, Glycerin, Sorbitan oder anderen Alkoholen entstehen,
4. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren
Vorteilhaft ist ferner die Verwendung einer Kombination von anionischen und/oder amphoteren Tensiden mit einem oder mehreren nicht-ionischen Tensiden.

### Antioxidantien

Es können die allgemein bekannten Antioxidantien Verwendung finden. Besonders bevorzugt sind jedoch Tocopherole und Derivate (z.B. Vitamin-E-acetat) sowie Butylhydroxytoluol und Butylhydroxyanisol.

### Vitamine

Es können die allgemein bekannten Vitamine, auch in Form ihrer Derivate und/oder Salze verwendet werden.

### Konservierungsstoffe

Die erfindungsgemäßen Zubereitungen können erfindungsgemäß vorteilhaft ein oder mehrere Konservierungsstoffe enthalten. Vorteilhafte Konservierungsstoffe im Sinne der vorliegenden Erfindung sind beispielsweise Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr.

Erfindungsgemäß vorteilhaft sind ein oder mehrere Konservierungsstoffe in einer Konzentration von 0,001 bis 2 Gew.-%, bevorzugt 0,01 bis 1,5 Gew.-% und besonders bevorzugt 0,05 bis 1 Gew.-%, jeweils bezogen auf das Gesamtgewicht der Zubereitung enthalten. Die Gewichtsangaben beziehen sich dabei auf die Zusammensetzung der Zubereitung vor der Trocknung.

### Farbstoffe und Pigmente

Es kann auch gegebenenfalls vorteilhaft im Sinne der vorliegenden Erfindung sein, den Zubereitungen gemäß der Erfindung Farbstoffe und/oder -pigmente einzuverleiben.

Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise nachfolgend aufgeführte: Die Colour Index Nummern (CIN) sind dem *Rowe Colour Index, 3. Auflage, Society of Dyers and Colourists, Bradford, England, 1971* entnommen.

| Chemische oder sonstige Bezeichnung | CIN | Farbe |
|---|---|---|
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| (N-Ethyl-p-sulfobenzyl-amino)-phenyl-(2-sulfophenyl)-methylen-(N-ethyl-N-p-sulfo-benzyl)Δ^{2,5}-cyclohexadienimin | 42090 | blau |
| Chinophthalon-disulfosäure | 47005 | gelb |

Bevorzugt können wasserlösliche Farbstoffe eingesetzt werden.

Es ist bei all diesem im Einzelfalle möglich, dass die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so dass er weiß, dass bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

### Beispiele für nachschäumende Rasiergele:

| Produktbezeichnung | Menge/% | Menge/% | Menge/% | Menge/% |
|---|---|---|---|---|
| Palmitinsäure | 8.90 | 7.00 | 9.30 | 4.00 |
| Stearinsäure | | 1.00 | | 4.00 |
| Triethanolamin | 7.00 | 5.5 | 7.15 | 6.5 |
| Polyethylenglykol(20)oleylether | 6.30 | 4.00 | 5.50 | 7.00 |
| Na-laurylmyristylethersulfat | | | 3.00 | 2.50 |
| Fettsäureamidoalkylbetain | | 2.00 | | 1.50 |
| Sorbitol | 4.20 | | 3.50 | |
| Glycerin | | 4.00 | | 5.00 |
| Propylene Glycol | | | 3,50 | |
| Kokosfettsäurediethanolamid | 1.05 | | | |
| Glycerinpolyglykol (90) ether Isostearat/Polyethylenglykol (2) laurylether¹ | | 0.50 | 1.00 | |
| Paraffinöl | 1.05 | 1.00 | 1.50 | |
| Polyisobuten | 0.53 | 0.30 | 1.00 | 0.85 |
| Hydroxyethylcellulose | 0.53 | 0.50 | 0.50 | 0.50 |
| PEG-14 M | 0.11 | 0.30 | 0.15 | |
| Isopentan | 3.95 | 3.95 | 3.95 | 3.95 |
| Isobutan | 1.30 | 1.30 | 1.30 | 1.30 |
| Propylparaben | 0.09 | q.s. | q.s. | q.s. |
| Methylparaben | 0.13 | q.s. | q.s. | q.s. |
| Butylhydroxytoluol | 0.03 | 0.03 | 0.03 | 0.03 |
| Farbstoff | q.s. | q.s. | q.s. | q.s. |
| Parfum | q.s. | q.s. | q.s. | q.s. |
| Wasser | add.100 | add.100 | add.100 | add.100 |
| Summe: | 100.00 | 100.00 | 100.00 | 100.00 |

| | | | | |
|---|---|---|---|---|
| 1 Glycerinisostearatpolyglykolether/Fettalkoholpolyglycolether 77%ig erhältlich unter dem Handelsnamen Oxetal VD 92 der Fa. Zschimmerer & Schwarz | | | | |

### Beispiele für Rasierschäume:

| Produktbezeichnung | Menge [%] | Menge [%] | Menge [%] | Menge [%] |
|---|---|---|---|---|
| Palmitinsäure | | 2,75 | 5,00 | 2,00 |
| Stearinsäure | 5,90 | 2,75 | | 4,00 |
| Glycerin | 3,75 | 2,00 | 5,00 | 4,00 |
| Na-laurylmyristylethersulfat (25%) | 3,60 | 5,00 | | 1.80 |
| Fettsäureamidoalkylbetain | | 1.50 | 2.70 | 1,90 |
| Triethanolamin | 3,50 | 3,80 | 3,10 | 4,15 |
| Ethylenglycolmonolaurylether [Polyethylenglykol (23) laurylether] | 2,95 | 2,75 | 5,00 | 4,00 |
| Coco-Caprylate/Caprate | | | | 0,50 |
| Glycerinpolyglykol (90) ether Isostearat/Polyethylenglykol (2) laurylether¹ | | 0,10 | 0,5 | |
| Paraffinöl | | 1,00 | | |
| Hydroxypropyl Methylcellulose | | | 0,15 | |
| Dimethylsiloxanglykolcopolymer | | 0,50 | 0,20 | |
| Stearylalkohol | | 0,10 | | 0,50 |
| Propylparaben | q.s. | q.s. | q.s. | q.s. |
| Isobutan | 2,90 | 2,90 | 2,90 | 2,90 |
| Propan | 0,95 | 0,95 | 0,95 | 0,95 |
| Butan | 0,20 | 0,20 | 0,20 | 0,20 |
| Methylparaben | q.s. | q.s. | q.s. | q.s. |
| Parfum | q.s. | q.s. | q.s. | q.s. |
| Aqua | add.100 | add.100 | add.100 | add.100 |
| Summe: | 100,00 | 100,00 | 100,00 | 100,00 |

## Patentansprüche

1. Seifenhaltige Öl-in-Wasser Emulsionen, die frei von Fettsäurediethanolamiden und zur Abfüllung in Aerosolverpackungen geeignet sind.
(a) 4 bis 12 Gew.% Fettsäuren mit 12 bis 18 Kohlenstoffatomen,
(b) 3 bis 8 Gew.% Trialkanolaminen sowie als weitere Komponenten,
(c) 1 bis 8 Gew.%, bevorzugt 2 bis 6 Gew.% eines nichtionischen Emulgators
(d) 0 bis 3 Gew. % eines Fettalkohols oder Fettalkoholgemisches,
(e) 0,1 bis 6 Gew.%, besonders bevorzugt 0,5 bis 5 Gew.% eines oder mehrerer Emollientien
(f) 1 bis 10, besonders bevorzugt 2 bis 5 Gew.% Treibgas mit einem Dampfdruck von 76 bis 420 kPa bei 20°C.
(g) 0 bis 1 Gew.%, bevorzugt 0,1 bis 1 Gew.% eines Cellulosederivates und
(h) 0,1 bis 4, besonders bevorzugt bis 3, besonders bevorzugt bis 2 Gew.% Glycerinpolyglycoletherisostearate, besonders bevorzugt solches mit 70 bis 110 Ethylenoxyd-Einheiten,
jeweils bezogen auf das Gesamtgewicht der Zubereitung, aufweisen.

2. Zubereitungen nach einem der vorangehenden Ansprüche **dadurch gekennzeichnet, dass** der oder die nichtionischen Emulgatoren einen HLB-Wert von mindestens 14 aufweisen.

3. Zubereitungen nach einem der vorangehenden Ansprüche **dadurch gekennzeichnet, dass** der oder die Emulgatoren gewählt werden aus der Gruppe der Fettsäurealkoxylate mit 10 bis 40, besonders bevorzugt 15 bis 30 Alkoxyeinheiten.

4. Zubereitungen nach einem der vorangehenden Ansprüche **dadurch gekennzeichnet, dass** das oder die Cellulosederivate gewählt werden aus der Gruppe HydroxypropylMethylcellulose, Hydroxyethylcellulose, Hydroxypropylcellulose.

5. Zubereitungen nach einem der vorangehenden Ansprüche **dadurch gekennzeichnet, dass** als Glycerinpolyglycoletherisostearat das Glycerinpolyglycolether-90-isostearat verwendet wird.

6. Zubereitungen nach einem der vorangehenden Ansprüche **dadurch gekennzeichnet, dass** sie als aus einer Druckgaspackung entnehmbare Schäume vorliegen.

7. Zubereitungen nach einem der vorangehenden Ansprüche **dadurch gekennzeichnet, dass** sie als aus einer Druckgaspackung entnehmbare Rasiergele vorliegen.

8. Zubereitungen nach Anspruch 8 **dadurch gekennzeichnet, dass** die Druckgaspackung zwei Kammern aufweist.

9. Packmittel enthaltend Zubereitungen nach einem der vorangehenden Ansprüche.
